# FASCICULE DE BREVET EUROPEEN

(11) **EP 0 900 061 B1**
(45) Date de publication et mention de la délivrance du brevet: **21.06.2000**
(21) Numéro de dépôt: 97919210.1
(22) Date de dépôt: 25.04.1997
(51) Int. Cl.: A61F 2/06

(54) **ENDOPROTHESE LUMINALE POUR RAMIFICATION DE VOIES D'UN CORPS HUMAIN OU ANIMAL ET SON PROCEDE DE FABRICATION**
ENDOLUMINALE PROTHESE ZUR KANALVERZWEIGUNG IN EINEM MENSCHLICHEN ODER TIERISCHEN KÖRPER SOWIE ENTSPRECHENDES HERSTELLUNGSVERFAHREN
INTRALUMINAL ENDOPROSTHESIS FOR RAMIFYING THE DUCTS OF A HUMAN OR ANIMAL BODY AND METHOD OF MANUFACTURE THEREOF

(30) Priorité: 25.04.1996 BE 9600364
(43) Date de publication de la demande: 10.03.1999
(73) Titulaire: CORVITA EUROPE, 1070 Bruxelles (BE); Dereume, Jean-Pierre, George, Emile, B-1050 Bruxelles (BE)
(72) Inventeur: DEREUME, Jean-Pierre, Georges, Emile, B-1050 Bruxelles (BE); FRID, Noureddine, B-1650 Beersel (BE)
(74) Mandataire: Claeys, Pierre
(86) Numéro de dépôt international: BE9700049
(87) Numéro de publication internationale: WO9740779

(56) Documents cités:
- EP-A- 0 551 179
- EP-A- 0 686 379
- FR-A- 2 678 508
- US-A- 5 507 769

## Description

La présente invention concerne une endoprothèse luminale pour ramification de voies d'un corps humain ou animal. Elle est aussi relative à un élément de tronc tubulaire à mettre en oeuvre dans une telle endoprothèse luminale et au procédé de fabrication d'un tel élément de tronc tubulaire.

On tente depuis longtemps de porter remède à diverses altérations qui apparaissent dans les parois des voies du corps humain ou animal, comme les vaisseaux sanguins, les conduits du système gastro-intestinal, les conduits urinaires et autres. Comme altérations très fréquentes de ce genre on peut citer les anévrismes de vaisseaux sanguins, en particulier les anévrismes qui frappent les segments sous-rénaux des aortes abdominales. Là l'aorte subit une dilatation entraînant un risque de rupture de la paroi vasculaire et le décès du patient.

Par l'utilisation d'endoprothèses luminales on a cherché à assister les parois vasculaires déficientes. Différents types d'endoprothèses sont décrits dans ce but dans la littérature, et notamment dans les brevets US-A-4140126 et 4512332 et dans les demandes de brevet internationales PCT WO 94/01056 et WO 96/07371. On peut mentionner également PARODI J.C. et al, Transfemoral Intraluminal Graft Implantation for Abdominal Aortic Aneurysms, Annals of Vascular Surgery, Vol. 5, n° 6-1991, p. 491-499.

Dans ces documents on décrit une fixation d'une endoprothèse aux deux collets distal et proximal de l'anévrisme, par exemple aortique abdominal, c'est-à-dire aux parties qui, au début de l'affection, sont le moins touchées. Le collet proximal se situe sous les artères rénales et le collet distal juste en amont de la bifurcation aorto-iliaque. Aucune solution n'est toutefois proposée lorsque l'anévrisme se prolonge dans les branches de cette ramification. En effet, au fur et à mesure de la croissance exponentielle de l'anévrisme, les collets deviennent aussi le siège d'une dilatation, particulièrement le collet distal, puis les artères iliaques. Dans de tels cas, très fréquents, les solutions proposées ci-dessus sont insuffisantes.

On a par conséquent cherché à réaliser des endoprothèses luminales pour ramification.

On connaît par exemple des endoprothèses bifurquées ayant l'allure générale d'un pantalon présentant aux trois extrémités un élément de tuteur d'accrochage, à fixer au collet proximal de l'anévrisme et dans des segments sains des artères iliaques (voir EP-A-0461791 et EP-A-0539237; T. CHUTER, Bifurcated endovascular graft insertion for abdominal aortic aneurysm, in "Vascular and Endovascular Surgical Techniques", 3e éd., Ed. RM Greenhalg Publication WD Saunders Company, 1994, p. 92-99). Ces endoprothèses présentent l'inconvénient d'un placement difficile, après le largage aortique, pour l'introduction de chacune des jambes de l'endoprothèse dans leur artère iliaque. On doit généralement faire appel à des dispositifs de récupération de cathéter croisé fémoral ce qui demande une grande maîtrise de la part du chirurgien.

D'autres endoprothèses bifurquées connues comprennent un segment aortique prolongé par un segment iliaque. Un moignon d'embranchement de 5 mm de diamètre fait saillie latéralement, et doit être placé en face de l'embouchure de l'artère iliaque non encore pourvue de son endoprothèse. Par cette artère iliaque, il faut alors introduire un tube additionnel à insérer dans ce petit moignon tubulaire. Cela nécessite un positionnement précis de celui-ci, ce qu'on tente d'obtenir par l'agencement de marqueurs radioopaques sur l'endoprothèse (voir BLUM U. et al, Abdominal Aortic Aneurysms...., International Radiology, Vol. 198, 1, January 1996, p. 25-31). De même que dans la solution précédente, il faut une grande maîtrise du chirurgien pour introduire le tube additionnel dans le moignon tubulaire qui lui est destiné et il doit posséder une grande expertise dans le domaine de l'utilisation des cathéters endoluminaux.

Toutes ces formes de réalisation, que ce soit celles bifurquées avec un tronc et deux jambes entières ou avec un tronc, une jambe entière et une jambe à insérer sur place dans un moignon, présentent l'inconvénient d'une forme compliquée. En outre, elles sont généralement supportées de manière rigide par des éléments de tuteur d'accrochage, uniquement au collet proximal et dans les artères iliaques, d'où le danger d'une coudure trop accentuée d'une des deux jambes à l'endroit de la bifurcation.

Pour tenter de surmonter ce dernier inconvénient on a prévu des tuteurs expansibles et rétractables qui peuvent soutenir un recouvrement lui aussi expansible, sur toute la longueur de l'endoprothèse bifurquée. Un modèle de tuteur bifurqué, expansible par ballon, a été prévu par exemple dans le brevet US-A-4994071. Il apparaît toutefois clairement que la fabrication d'une endoprothèse bifurquée munie d'un tuteur bifurqué complet est complexe et coûteuse. Son introduction dans le corps du patient n'est certainement pas aisée non plus.

On connaît enfin des endoprothèses formées de deux éléments tubulaires à introduire simultanément dans l'anévrisme, le premier par une artère iliaque, le second par l'autre artère iliaque. Les extrémités de ces éléments tubulaires, lorsqu'elles sont parvenues dans le collet proximal de l'anévrisme, sont dilatées radialement pour y être accrochées simultanément, tandis que l'extrémité opposée de chacun de ces éléments est accrochée de manière semblable dans son artère iliaque correspondante. Suivant une forme de réalisation, préalablement à ces opérations, un tuteur métallique est dilaté dans le collet pour recevoir les deux extrémités dilatables des éléments tubulaires précités (voir EP-A-0551179).

Ces formes de réalisation présentent l'inconvénient majeur de ne pas garantir une étanchéité parfaite des deux extrémités proximales des éléments tubulaires à l'intérieur du collet proximal de l'anévrisme. Il s'ensuit inévitablement des fuites à la périphérie des éléments tubulaires, une pénétration de sang dans l'anévrisme et le rétablissement, à éviter, de la pression sanguine à cet endroit.

La présente invention a pour but de surmonter les problèmes posés et de proposer une endoprothèse luminale pour ramification de voies d'un corps humain ou animal qui soit applicable à la majorité des conditions anatomiques et qui soit facile à positionner sans disposer d'une expérience particulière du cathétérisme. Après placement, cette endoprothèse ne peut présenter aucun phénomène de fuite de sang dans la cavité de l'anévrisme. Cette endoprothèse sera de plus avantageusement très aisée à fabriquer et à stocker.

On résout ce problème par une endoprothèse luminale pour ramification de voies d'un corps humain ou animal, comprenant
- un élément de tronc tubulaire, radialement expansible et compressible, à appliquer en position d'expansion dans une voie principale de ladite ramification, cet élément de tronc tubulaire présentant axialement deux extrémités et une cavité qui est ouverte à ces deux extrémités et
- au moins un élément d'embranchement tubulaire, radialement expansible et compressible, présentant axialement deux extrémités et une cavité ouverte à ces deux extrémités, chaque élément d'embranchement étant, dans sa position de compression, indépendant de l'élément de tronc tubulaire,
cette endoprothèse étant caractérisée en ce que la cavité de l'élément de tronc est divisée en plusieurs chenaux axiaux sur au moins une partie de sa longueur, et en ce que chaque élément d'embranchement présente une extrémité à appliquer, en position d'expansion, dans un desdits chenaux axiaux de l'élément de tronc tubulaire et une autre extrémité située à l'extérieur de l'élément de tronc tubulaire, dans une voie secondaire de ladite ramification.

Cette endoprothèse offre l'avantage d'être formée d'éléments indépendants, c'est-à-dire à introduire successivement dans le corps, qui ont une forme extérieure semblable aux endoprothèses tubulaires courantes connues. L'élément de tronc est largué entre les collets distal et proximal de l'anévrisme d'une manière courante, comme une endoprothèse non bifurquée. Ensuite, chaque élément d'embranchement est introduit par une extrémité à l'intérieur de l'élément de tronc, en restant à l'autre extrémité dans son artère iliaque correspondante. La division de la cavité interne de l'élément de tronc tubulaire en deux chenaux axiaux a pour effet de déplacer artificiellement l'embranchement à une certaine distance de l'embranchement altéré. L'application d'un élément d'embranchement tubulaire dans chaque chenal axial a pour effet d'empêcher tout phénomène de fuite de sang dans l'anévrisme.

Avantageusement, les chenaux axiaux sont prévus uniquement sur une partie centrale de la cavité axiale de l'élément de tronc tubulaire. Cette forme de réalisation facilite, comme on le verra dans la suite, l'introduction du guide et de son introducteur, puis de son élément d'embranchement à l'intérieur de l'élément de tronc déjà placé. De plus, la bifurcation obtenue par deux éléments d'embranchement, accrochés au centre de l'élément de tronc, adoucit la courbure des éléments d'embranchement, ce qui favorise l'écoulement des fluides corporels.

Suivant une forme de réalisation avantageuse, l'élément de tronc tubulaire comprend entre ses extrémités un manchon en matière souple, biocompatible, qui est imperméable aux fluides corporels passant dans ladite ramification et qui forme ladite cavité et ses chenaux, et, au moins à chaque extrémité de l'élément de tronc, un élément de tuteur tubulaire, radialement expansible et compressible, auquel le manchon est fixé. Suivant une forme perfectionnée de réalisation de l'invention, l'élément de tronc tubulaire comprend, entre ses extrémités, un manchon en matière souple, biocompatible, qui est imperméable aux fluides corporels passant dans ladite ramification, et qui forme ladite cavité et ses chenaux, et un tuteur tubulaire radialement expansible et compressible, qui entoure le manchon et sur lequel celui-ci est fixé au moins aux extrémités de l'élément de tronc tubulaire. Il n'est donc pas besoin d'envisager de tuteur bifurqué. Il suffit d'utiliser des tuteurs tubulaires, non bifurqués, aisés à réaliser et connus depuis longtemps dans la technique. De tels tuteurs sont par exemple autoexpansibles ou expansibles par ballon et on peut citer à titre de référence par exemple les US-A-4733655, US-A-4739762, US-A-4776337, US-A-5019090, US-A-5061275, US-A-5092877, US-A-5171262, US-A-5195984, EP-A-0183372, EP-A-0556850, EP-A-0621015, GB-1205743, WO-83/03752, WO-92/06734.

Le manchon peut être réalisé en n'importe quelle matière biocompatible connue qui a par exemple déjà été utilisée pour la fabrication de greffes ou de recouvrements de tuteur d'endoprothèses. On peut citer notamment une matière produite par exemple comme décrit dans le US-A-4475272, le US-A-4323525 ou le EP-A-0603959. Cette matière biologiquement inerte peut être du DACRON, du TEFLON, du polyuréthanne, des fibres de polycarbonate, ou des matières analogues.

Avantageusement le tuteur tubulaire expansible présentera, en position d'expansion, au moins une extrémité qui s'évase vers l'extérieur. De préférence il présentera un recouvrement complet interne et/ou externe.

Chaque élément d'embranchement est réalisé sous la forme d'une endoprothèse connue en soi appropriée pour être placée à l'intérieur d'une voie secondaire de ramification, par exemple d'une artère iliaque.

Suivant une forme de réalisation de l'invention, chaque élément d'embranchement tubulaire comprend, entre ses extrémités, une gaine en matière souple, biocompatible, qui est imperméable aux fluides corporels passant par ladite ramification et qui forme sa cavité susdite et, au moins à chaque extrémité de l'élément d'embranchement tubulaire, un élément de tuteur tubulaire, radialement expansible et compressible, sur lequel la gaine est fixée.

Suivant une forme avantageuse de réalisation de l'invention, chaque élément d'embranchement tubulaire comprend, entre ses extrémités, un tuteur tubulaire radialement expansible et compressible, qui présente un recouvrement interne et/ou externe en une matière biocompatible, imperméable aux fluides corporels. Dans ce cas, comme on peut le constater, l'endoprothèse peut être soutenue sur toute sa longueur par plusieurs tuteurs tubulaires, un tuteur pour l'élément de tronc et un tuteur pour chaque élément d'embranchement, aucun de ces tuteurs n'étant bifurqué.

La présente invention concerne aussi un élément de tronc tubulaire à mettre en oeuvre dans une endoprothèse luminale pour ramification de voies du corps humain ou animal, cet élément de tronc tubulaire étant radialement expansible et compressible et étant, en position d'expansion, à appliquer dans une voie principale de ladite ramification, l'élément de tronc tubulaire présentant axialement deux extrémités et une cavité qui est ouverte à ces deux extrémités et qui est divisée en plusieurs chenaux axiaux sur au moins une partie de sa longueur.

L'invention est également relative à des procédés de fabrication d'élément de tronc tubulaire suivant l'invention à mettre en oeuvre dans un endoprothèse luminale pour ramification de voies d'un corps humain ou animal.

Des formes ou modes de réalisation de l'invention sont indiqués dans les revendications qui suivent.

D'autres détails et particularités de l'invention ressortiront de la description donnée ci-après, à titre non limitatif et avec référence aux dessins annexés.

La figure 1 représente une vue en élévation latérale, partiellement brisée, d'un élément de tronc tubulaire suivant l'invention, à mettre en oeuvre dans une endoprothèse luminale suivant l'invention.

Les figures 2 à 4 représentent des vues en coupe, à l'échelle agrandie, suivant les lignes II-II, III-III et IV-IV de la figure 1.

La figure 5 représente une vue en élévation latérale, partiellement brisée, d'une variante de réalisation d'élément de tronc tubulaire suivant l'invention.

Les figures 6 à 8 représentent des vues en coupe, à l'échelle agrandie, suivant les lignes VI-VI, VII-VII et VIII-VIII de la figure 5.

La figures 9 à 14 illustrent, dans des vues partiellement brisées, un mode de largage d'une endoprothèse luminale pour ramification suivant l'invention dans le cas d'un anévrisme d'aorte abdominale.

Les figures 15 à 17 illustrent un mode de fabrication d'élément de tronc suivant l'invention.

Sur les différents dessins, les éléments identiques ou analogues sont désignés par les mêmes références.

Il faut par ailleurs noter que, sur les figures 2 à 4 et 6 à 8, les fils des tuteurs sont représentés en section, et non en coupe comme les autres éléments, pour faciliter la lecture des dessins. Sur ces vues les proportions entre les différents éléments ne sont pas nécessairement correctes. L'épaisseur des couches a par exemple été augmentée pour une meilleure compréhension des dessins.

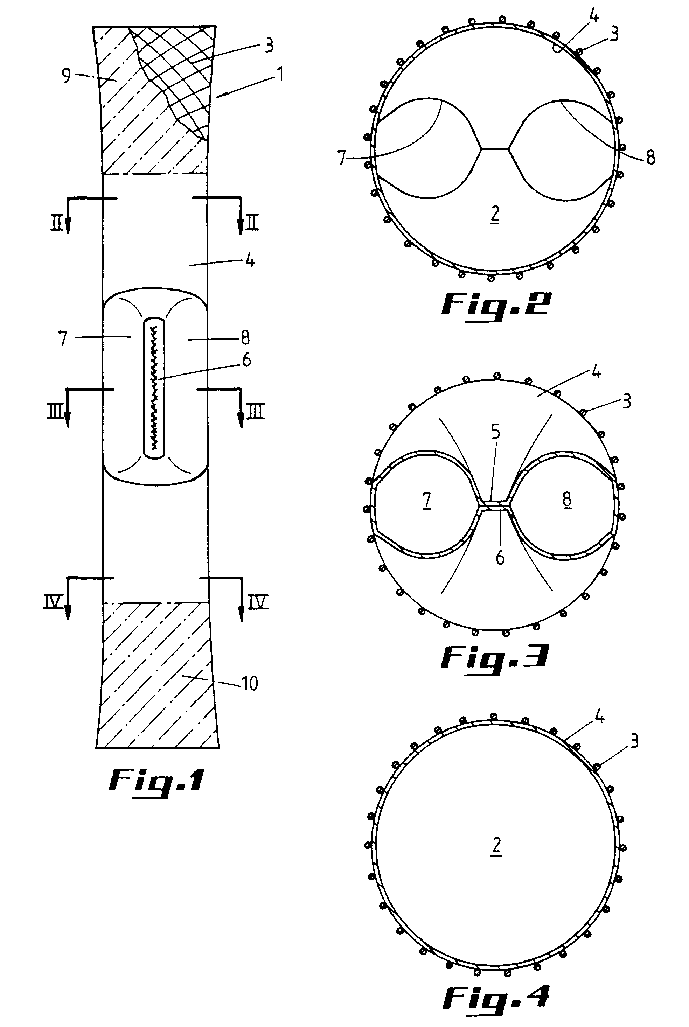

Sur les figures 1 à 4 on a représenté une forme de réalisation d'élément de tronc tubulaire, désignée par la référence générale 1, d'une endoprothèse pour ramification suivant l'invention.

Cet élément de tronc tubulaire est radialement expansible et compressible et il est représenté en position d'expansion. Il présente axialement deux extrémités et une cavité 2 qui est ouverte à ces deux extrémités.

Dans l'exemple illustré, l'élément de tronc tubulaire 1 comprend un tuteur tubulaire 3, connu en soi, ici formé d'une tresse tubulaire autoexpansible, par exemple à base de fils métallique simples, et un manchon 4 en une matière biocompatible, avantageusement souple, qui est imperméable aux fluides corporels passant dans ladite ramification.

Le manchon 4 présente, dans sa partie centrale, des parties diamétralement opposées 5 et 6 qui sont mutuellement reliées, par exemple par couture, scellage, thermoscellage, etc... Les extrémités du manchon sont fixées sur la paroi interne du tuteur 3, par exemple par collage.

Le manchon 4 délimite la cavité interne 2 de l'élément de tronc tubulaire et, par sa forme, il réalise à l'intérieur de ce dernier un agencement de deux chenaux axiaux 7 et 8 qui divisent la cavité 2 en deux conduits parallèles, qui sont de forme semblable à des conduits vasculaires et sont isolés de manière étanche l'un par rapport à l'autre et chacun par rapport à l'anévrisme.

Suivant une forme particulière de réalisation de l'invention, on peut imaginer que le tuteur 3 soit remplacé par des éléments de tuteur 9 et 10 aux extrémités de l'élément de tronc 1 pour l'accrochage du manchon 4 en place (voir figure 1 en traits interrompus). Le manchon 4 est fixé à ses extrémités à ces éléments de tuteur tandis qu'il n'est pas soutenu dans sa partie centrale. Sa forme rétrécie à cet endroit en deux chenaux axiaux 7 et 8 lui confère là une meilleure résistance à la pression interne radiale du fluide corporel traversant le manchon après le largage de l'élément de tronc tubulaire 1.

On peut aussi prévoir que le tuteur ou les éléments de tuteur soient à amener en position d'expansion à l'aide d'un ballon gonflable, d'une manière connue en soi.

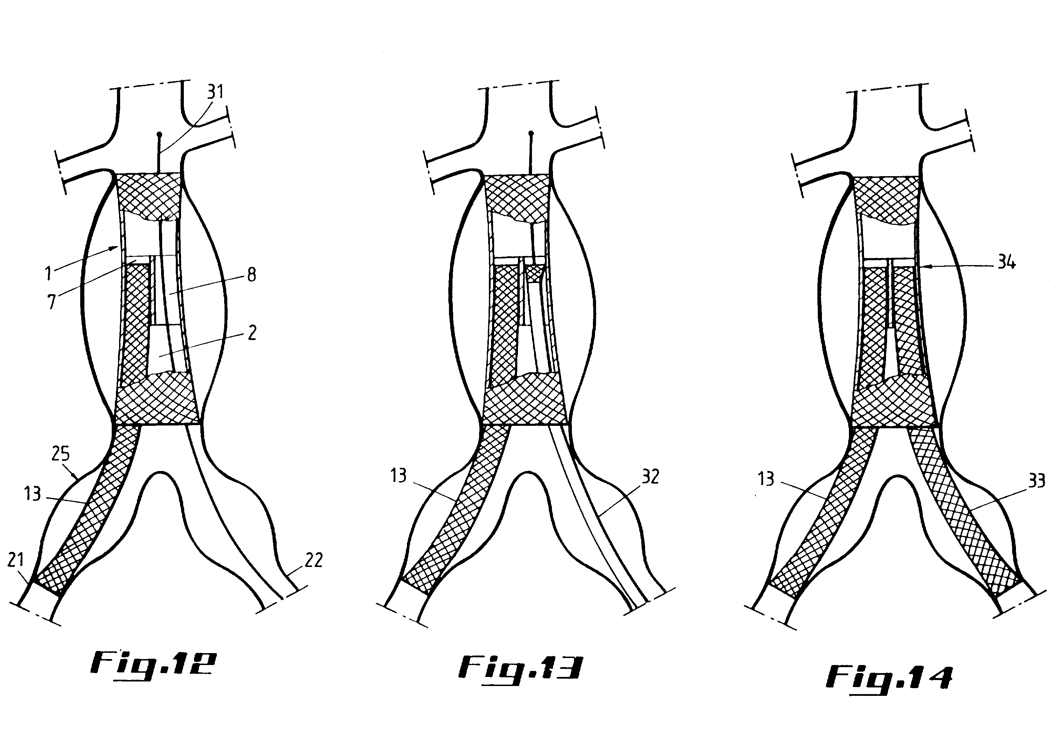

Avantageusement la tresse tubulaire du tuteur 3 illustré peut être formée de fils multiples. Elle présentera aussi de préférence, plutôt qu'une forme cylindrique, une forme à extrémités évasées, pour pouvoir être fixée plus aisément aux collets de l'anévrisme ainsi qu'on peut le voir sur les figures 13 à 18.

On peut aussi envisager une extension des deux chenaux 7 et 8 sur une plus grande longueur de l'élément de tronc 1.

Dans la forme de réalisation illustrée sur les figures 5 à 8, le tuteur 3 présente un recouvrement interne 11 et un recouvrement externe 12, par exemple en une matière biocompatible. Un tel tuteur est bien connu dans la technique, par exemple dans la EP-A-0603959. Les matières de ces recouvrements peuvent être les mêmes, et avantageusement le manchon 4 fixé à l'intérieur de ce tuteur est également lui-même dans cette matière.

Cette forme de réalisation permet une excellente adhésion du manchon 4 à deux chenaux 7 et 8 à l'intérieur du tuteur, ainsi qu'il sera expliqué de manière plus détaillée dans la suite.

Sur les figures 5 à 8, on a représenté, en place, un élément d'embranchement 13 suivant l'invention radialement rétractable et expansible. Cet élément d'embranchement tubulaire 13 présente axialement deux extrémités et une cavité 30 ouverte à ces extrémités. Une de ses extrémités est fixée dans le chenal 8. Dans l'exemple illustré, cet élément d'embranchement 13 comprend, comme une endoprothèse luminale courante, un tuteur tubulaire 14 connu en soi, formé ici d'une tresse tubulaire autoexpansible, par exemple à base de fils métalliques simples. Cette tresse présente ici un recouvrement externe 15 en une matière biocompatible, avantageusement souple, qui est imperméable aux fluides corporels passant dans la ramification.

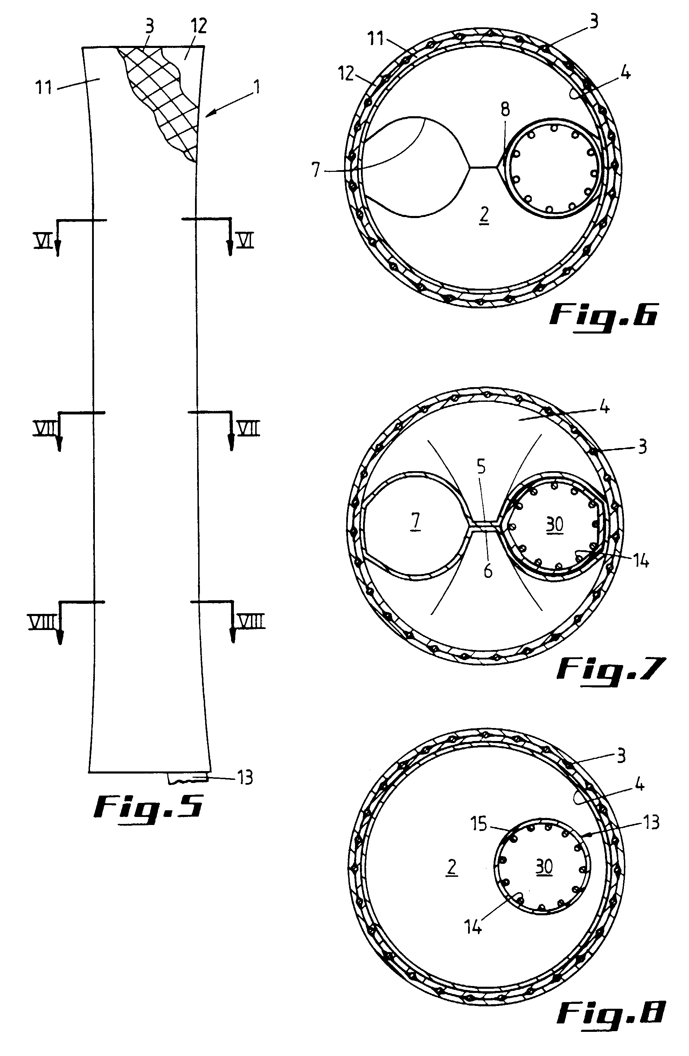

Comme on peut le voir sur la figure 7, dans sa position d'expansion dans le chenal 8, l'extrémité de l'élément d'embranchement 13 s'applique contre la paroi interne du chenal 8 d'une manière étanche aux fluides. Il peut présenter un recouvrement 15 en une matière identique à celle du ou des recouvrements du tuteur 3 et à celle du manchon 4. Cet élément d'embranchement 13 peut plutôt présenter un recouvrement interne au lieu du recouvrement externe 15 ou il peut comporter les deux à la fois.

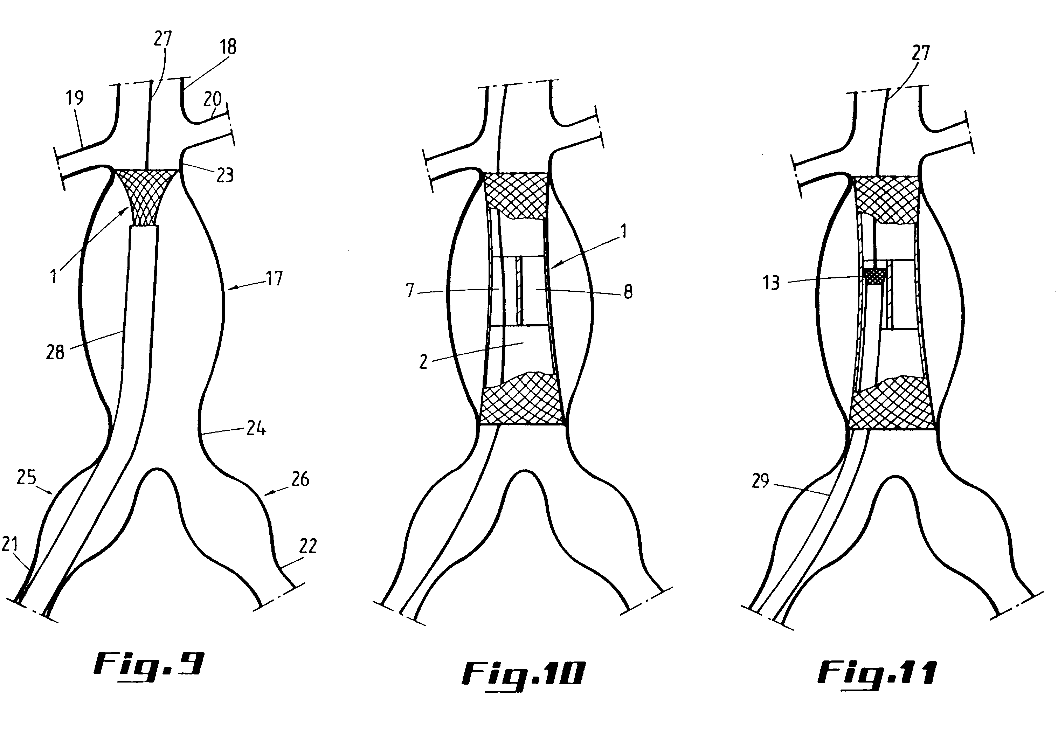

Ainsi qu'il ressort de la figure 8, l'élément d'embranchement 13, hors du chenal 8, flotte assez librement dans la cavité 2 de l'élément de tronc tubulaire 1. Il fait saillie à une extrémité de celui-ci (voir figure 5). C'est cette extrémité de l'élément d'embranchement 13 qui se trouvera à l'intérieur d'une artère iliaque, en cas d'anévrisme d'artère abdominale.

Dans cette position, l'élément d'embranchement 13 met en communication l'artère iliaque directement avec la partie haute sur la figure 5 de l'élément de tronc 1, c'est-à-dire la partie en amont des chenaux 7 et 8. Cette communication se fait d'une manière parfaitement étanche vis-à-vis du chenal 7 ne contenant pas encore d'élément embranchement (voir figure 7) et de la cavité 2 dans sa partie basse sur la figure 5 (voir figure 8).

Comme on le verra plus loin de manière plus détaillée, l'endoprothèse suivant l'invention réalise donc une bifurcation non anatomique entre des éléments d'endoprothèse séparés, qui sont introduits les uns dans les autres de manière étanche. L'extrémité de l'élément de tronc de l'endoprothèse par laquelle peuvent être introduits les éléments d'embranchement est largement ouverte et ne permet aucune fausse manoeuvre pour l'insertion de ceux-ci dans un des chenaux 7 et 8.

Il est entendu que d'autres formes de réalisation de l'élément de tronc 1 peuvent être conçues sans sortir du cadre de l'invention. On peut par exemple imaginer d'autres façons de cloisonner deux ou plusieurs chenaux de façon étanche l'un à l'autre. Une simple cloison souple à l'intérieur du tuteur (comme schématisé sur la figure 10) pourrait convenir. Un cloisonnement non étanche suffit pour autant que, lorsque les éléments d'embranchement sont introduits, l'étanchéité soit alors garantie par expansion de ceux-ci.

A l'aide des figures 9 à 14, on va à présent décrire l'introduction d'une endoprothèse suivant l'invention dans un anévrisme d'aorte abdominale. L'anévrisme 17 dans cette aorte abdominale 18 se situe entre les artères rénales 19 et 20 et les artères iliaques 21 et 22. L'anévrisme 17 présente deux collets, le proximal 23 et le distal 24. Le départ des artères iliaques présente chacun également un anévrisme 25 et 26.

D'une manière courante, on introduit à partir d'une des artères iliaques 21, un guide 27, c'est-à-dire un long fil qui va guider le coulissement d'un introducteur 28 à l'intérieur de l'artère iliaque, puis de l'artère aorte (voir figure 9). Lorsque l'introducteur 28 ainsi guidé est parvenu au niveau du collet proximal 23, on peut larguer l'élément de tronc 1 suivant l'invention, comme lors du largage d'une endoprothèse courante (voir par exemple US-A-4140126), par simple retrait de l'introducteur 28 et maintien en place de l'élément de tronc 1. Celui-ci, dans l'exemple illustré, s'évase alors sous l'action d'expansion du tuteur et, de cette manière, il s'applique sur le collet proximal 23 de manière à étanchéifier l'anévrisme 17 vis-à-vis du flux sanguin provenant du coeur.

Lorsque, ainsi qu'il est représenté sur la figure 10, l'introducteur est complètement retiré, l'élément de tronc 1, dont la longueur a été calculée par le chirurgien, est en place avec chacune de ses extrémités appliquée sur un collet de l'anévrisme. Celui-ci est maintenant complètement isolé du flux sanguin passant par l'aorte abdominale.

On introduit alors un nouvel introducteur 29 le long du guide 27 qui est toujours en place. Cet introducteur pénètre sans problème dans la cavité 2 de l'élément de tronc 1. Dans une position comprimée à l'intérieur de l'introducteur 29, un élément d'embranchement 13 se trouve là dans un état totalement indépendant de l'élément de tronc 1. Lorsque, comme représenté sur la figure 11, l'extrémité de l'introducteur 29 parvient aisément dans l'un des chenaux 7 de l'élément de tronc 1, puis qu'on retire l'introducteur 29 en maintenant en place l'élément d'embranchement 13, l'extrémité de celui-ci passe en position d'expansion. Il s'applique alors contre les parois du chenal 7 où passe le guide 27.

Ainsi qu'il ressort de la figure 12, après retrait complet de l'introducteur 29, l'autre extrémité de l'élément d'embranchement 13, dont la longueur appropriée a été calculée par le chirurgien, s'applique contre un segment sain de l'artère iliaque, au-delà de l'anévrisme 25.

On introduit alors un nouveau guide 31 par l'autre artère iliaque 22. Ce guide pénètre sans problème, par la large ouverture de sa cavité 2, à l'intérieur de l'élément de tronc 1. Là il ne peut passer que par le chenal 8, le chenal 7 étant déjà obturé vis-à-vis de la partie basse de l'élément de tronc. Même, si à l'inverse de ce qui est représenté, l'élément d'embranchement 13 était placé entre l'artère iliaque 21 et le chenal 8, ce qui est possible, le guide inséré par l'artère iliaque 22 n'aurait que la possibilité de passer par le chenal non obturé, dans ce cas le chenal 7. On obtiendrait en fait une disposition finale croisée des éléments d'embranchement ce qui ne poserait aucun problème pour l'écoulement du sang.

Dans l'exemple illustré sur la figure 13, un nouvel introducteur 32 est alors glissé le long de son guide 31 et un élément d'embranchement 33 supplémentaire est alors largué comme représenté sur la figure 14.

Comme on peut le voir sur cette figure, l'embranchement anatomique a été rehaussé jusqu'au niveau indiqué par la flèche 34. Les éléments d'embranchement 13 et 33 présentent à partir de ce niveau une courbure douce sans danger de formation de pli. Les 3 anévrismes sont à présent étanchéifiés, sans avoir dû faire appel à des manoeuvres compliquées à l'aide de dispositif de récupération de cathéter ou à un alignement difficile de morceaux d'endoprothèse l'un en face de l'autre.

On peut imaginer une opération de placement de l'endoprothèse quelque peu différente. Par exemple, après l'étape illustrée sur la figure 10, on peut introduire sur le guide 27 un ballon gonflable connu en soi pour obturer le chenal 7. On introduit alors le guide 31 qui se trouve dans l'obligation de passer dans le chenal 8. On retire alors le ballon gonflable tout en maintenant son guide en place. On introduit ensuite simultanément à partir des deux artères iliaques les deux introducteurs 29 et 32 qui vont passer chacun, sans problème et simultanément, dans leur chenal approprié.

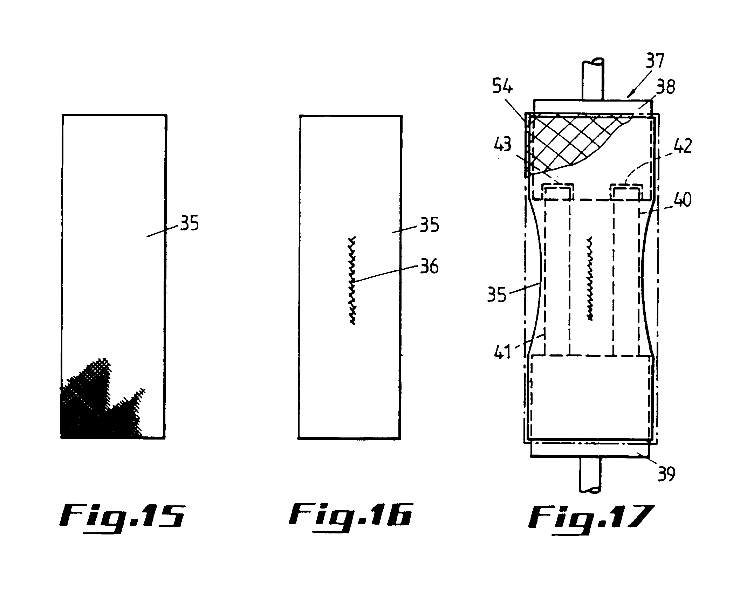

Pour fabriquer une endoprothèse luminale pour ramification de voies d'un corps humain ou animal suivant l'invention, on peut prévoir l'assemblage d'un manchon interne en matière souple, biocompatible, à un tuteur ou à des éléments de tuteur. Comme on l'a déjà dit, ceux-ci sont bien connus dans la technique. Au départ, le manchon est fabriqué sous une forme cylindrique d'une manière connue quelconque, par exemple selon l'enseignement des US-A-4323525, US-A-4475972, US-A-4878908 et de la EP-A-0009941. La matière utilisée pour fabriquer un tel manchon peut consister, bien que non nécessairement, en une matière fibreuse, avantageusement élastique. On peut citer, comme exemple de fibres très appropriées, des fibres de polycarbonate mises sur le marché par la firme CORVITA Corp. sous la dénomination de CORETHANE®. Le manchon cylindrique 35 obtenu, illustré sur la figure 15, subit ensuite une liaison étanche 36 entre deux parties diamétralement opposées de sa périphérie de façon à former deux chenaux axiaux sur au moins une partie de sa longueur. Cette liaison étanche peut par exemple être réalisée par couture, suture, scellage à chaud ou à froid ou tout autre moyen analogue.

Le manchon ainsi formé est alors à attacher sur la surface interne du tuteur tubulaire ou des éléments de tuteur utilisés par tout moyen approprié.

Pour cette étape d'attachement on peut procéder de la manière suivante : on utilise un moule approrié, désigné par la référence 37 sur la figure 17. Ce moule 37 comprend deux extrémités cylindriques 38 et 39 séparables. L'une de ces extrémités, 39 par exemple, est pourvue de deux jambes 40 et 41, qui font saillie parallèlement l'une à l'autre et à l'axe du moule 37. Après enfilage d'une partie du manchon 35 sur cette extrémité 39 du moule, en faisant passer les jambes 40 et 41 à travers les chenaux axiaux du manchon 35, on enfile la partie restante du manchon sur l'autre extrémité 38 du moule 37. Par rapprochement des deux parties de moule 38 et 39, les deux jambes 40 et 41 de la partie 39 pénètrent dans des trous borgnes 42 et 43 correspondants prévus dans la partie 38.

Dans cette position, le manchon 35 subit une expansion radiale. On peut alors appliquer à sa surface un adhésif et enfiler alors, par-dessus le manchon, le tuteur 54 ou les éléments de tuteur en position radialement expansée, à un diamètre légèrement supérieur au diamètre de repos. Le tuteur applique alors une pression dirigée vers l'intérieur sur le manchon, pendant que la colle prend.

Comme adhésif on peut envisager par exemple la matière dont le manchon est lui-même formé. Dans le cas de fibres de polycarbonate, on peut préparer une solution de cette matière dans un solvant, tel que du tétrahydrofuranne. Le tuteur est immergé dans cette solution avant son enfilage par-dessus le manchon. Après chauffage dans un four à air chaud à 110°C pendant une ½ heure, un élément de tronc suivant l'invention est obtenu.

On peut par exemple aussi appliquer des fibres mouillées de polycarbonate sur le manchon 35 enfilé sur le moule 37, selon l'enseignement donné dans la EP-A-0603959.

Lors de l'enfilage du tuteur immergé dans la solution adhésive précitée par-dessus le moule, les fibres mouillées très fines qui viennent d'être appliquées à la surface du manchon vont jouer le rôle de pont entre le film présenté sur la surface interne du tuteur et le manchon. On obtient une adhésion polymère-polymère, les deux polymères étant de même nature.

Suivant un mode de réalisation préféré, le tuteur présente avant son enfilage un recouvrement interne en fibres de polycarbonate et encore plus avantageusement, simultanément, un recouvrement externe, comme les tuteurs décrits dans la EP-A-0603959.

Le niveau d'énergie d'adhésion obtenu entre le manchon et le tuteur peut alors être de 100 à 1000 J/m².

Il doit être entendu que la présente invention n'est en aucune façon limitée aux formes et modes de réalisation donnés ci-dessus et que bien des modifications peuvent y être opportées sans sortir du cadre de l'invention indiquée dans les revendications qui suivent.

## Revendications

1. Elément de tronc (1) tubulaire d'endroprothèse luminale pour ramification de voies du corps humain ou animal, qui est radialement expansible et compressible et est, en position d'expansion, à appliquer dans une voie principale de ladite ramification,
caractérisé en ce qu'il comporte une cavité (2) qui s'étend, suivant un axe longitudinal, entre deux extrémités de l'élément de tronc (1), ladite cavité (2) étant ouverte à une première desdites deux extrémités par une première ouverture et à une deuxième desdites deux extrémités par une deuxième ouverture et étant divisée axialement et d'une manière imperméable aux fluides corporels, sur au moins une partie de sa longueur, de façon à former ainsi plusieurs chenaux axiaux (7, 8) qui s'étendent chacun suivant un axe parallèle audit axe longitudinal.

2. Elément de tronc d'endoprothèse suivant la revendication 1, caractérisé en ce qu'il comporte lesdits chenaux axiaux (7, 8) uniquement sur une partie centrale de sa cavité (2).

3. Elément de tronc d'endoprothèse suivant l'une des revendications 1 et 2, caractérisé en ce qu'il comporte deux chenaux axiaux (7, 8).

4. Elément de tronc d'endoprothèse suivant l'une des revendications 1 à 3, caractérisé en ce qu'il comprend entre ses extrémités un manchon (4, 35) en matière souple, biocompatible, qui est imperméable aux fluides corporels passant dans ladite ramification et qui forme ladite cavité (2) et ses chenaux (7, 8), et, au moins à chaque extrémité de l'élément de tronc (1), un élément de tuteur (9, 10) tubulaire, radialement expansible et compressible, auquel le manchon (4, 35) est fixé.

5. Elément de tronc d'endoprothèse suivant l'une des revendications 1 à 3, caractérisé en ce qu'il comprend, entre ses extrémités, un manchon (4, 35) en matière souple, biocompatible, qui est imperméable aux fluides corporels passant dans ladite ramification, et qui forme ladite cavité (2) et ses chenaux (7, 8), et un tuteur tubulaire (3, 54) radialement expansible et compressible, qui entoure le manchon (4, 35) et sur lequel celui-ci est fixé au moins aux extrémités de l'élément de tronc (1) tubulaire.

6. Elément de tronc d'endoprothèse suivant l'une des revendications 4 et 5, caractérisé en ce que le manchon (4, 35) présente des parties diamétralement opposées (5, 6) qui sont mutuellement reliées d'une manière étanche aux liquides corporels sur au moins une partie de la longueur du manchon (4, 35), en formant ainsi deux chenaux (7, 8) à l'intérieur du manchon.

7. Elément de tronc d'endoprothèse suivant l'une des revendications 4 à 6, caractérisé en ce que le manchon (4, 35) fixé sur le tuteur tubulaire (3, 54) ou le ou les éléments de tuteur tubulaires (9, 10) forme un recouvrement interne direct sur au moins une partie de celui-ci ou de ceux-ci.

8. Elément de tronc d'endoprothèse suivant l'une des revendications 4 à 6, caractérisé en ce que le tuteur tubulaire (3, 54), radialement expansible et compressible, ou chaque élément de tuteur tubulaire (9, 10), radialement expansible et compressible, comprend un recouvrement interne (11) en une matière biocompatible inerte, imperméable aux fluides corporels passant dans ladite ramification, et en ce que le manchon (4, 35) est fixé au tuteur (3, 54) ou aux éléments de tuteur (9, 10) par l'intermédiaire de ce recouvrement interne.

9. Elément de tronc d'endoprothèse suivant l'une des revendications 1 à 3, caractérisé en ce qu'il comprend entre ses extrémités, un tuteur tubulaire (3, 54), radialement expansible ou compressible, qui présente un recouvrement (11, 12) en une matière biocompatible, imperméable aux fluides corporels passant dans ladite ramification, en ce que le tuteur tubulaire (3, 54) recouvert forme ladite cavité (2) et en ce que l'élément de tronc (1) tubulaire comprend en outre au moins un élément de cloison souple, fixé dans ladite cavité (2) de manière à diviser celle-ci sur au moins une partie de sa longueur en lesdits plusieurs chenaux axiaux (7, 8).

10. Elément de tronc d'endoprothèse suivant l'une quelconque des revendications 4 à 9, caractérisé en ce que le tuteur tubulaire (3, 54) ou respectivement les éléments de tuteur tubulaires (9, 10) présentent un recouvrement interne (11) et/ou externe (12) en une matière biocompatible, imperméable aux fluides corporels.

11. Elément de tronc d'endoprothèse suivant l'une des revendications 5 à 10, caractérisé en ce que le tuteur tubulaire (3, 54) expansible présente, en position d'expansion, au moins une extrémité qui s'évase vers l'extérieur.

12. Endoprothèse luminale pour ramification de voies d'un corps humain ou animal, comprenant un élément de tronc (1) tubulaire suivant l'une quelconque des revendications 1 à 11, ainsi qu'au moins un élément d'embranchement (13, 33) tubulaire, radialement expansible et compressible, présentant axialement deux extrémités et une cavité (30) ouverte à ces deux extrémités, chaque élément d'embranchement étant, dans sa position de compression, indépendant de l'élément de tronc (1) tubulaire, et présentant une extrémité à appliquer, en position d'expansion, dans un desdits chenaux axiaux (7, 8) de l'élément de tronc tubulaire et une autre extrémité située à l'extérieur de l'élément de tronc (1) tubulaire, dans une voie secondaire de ladite ramification.

13. Endoprothèse suivant la revendications 12, caractérisée en ce que chaque élément d'embranchement (13, 33) tubulaire comprend entre ses extrémités, une gaine en matière souple, biocompatible, qui est imperméable aux fluides corporels passant par ladite ramification et qui forme sa cavité (30) susdite et, au moins à chaque extrémité de l'élément d'embranchement tubulaire, un élément de tuteur tubulaire, radialement expansible et compressible, sur lequel la gaine est fixée.

14. Endoprothèse suivant l'une des revendications 12 et 13, caractérisée en ce que chaque élément d'embranchement (13, 33) tubulaire comprend, entre ses extrémités, un tuteur tubulaire (14) radialement expansible et compressible, qui présente un recouvrement interne et/ou externe (15) en une matière biocompatible, imperméable aux fluides corporels.

15. Endoprothèse suivant l'une quelconque des revendications 12 à 14, caractérisée en ce que les chenaux (7, 8) de l'élément de tronc tubulaire ont une section circulaire d'un diamètre approximativement égal à un diamètre de voie secondaire de ladite ramification.

16. Procédé de fabrication d'un élément de tronc tubulaire radialement expansible et compressible à mettre en oeuvre dans une endoprothèse luminale pour ramification de voies d'un corps humain ou animal, comprenant
- une formation d'un manchon tubulaire en matière souple, biocompatible, qui est imperméable aux fluides corporels passant dans ladite ramification, et qui présente une cavité qui s'étend suivant un axe longitudinal entre deux extrémités ouvertes du manchon,
- une division axiale de ladite cavité, sur au moins une partie de sa longueur, en reliant de manière imperméable aux fluides corporels deux parties périphériques opposées du manchon, de façon à former deux chenaux axiaux,
- un attachement de ce manchon sur au moins une partie de sa longueur, à un tuteur tubulaire radialement expansible et compressible ou à au moins un élément de tuteur tubulaire radialement expansible et compressible.

17. Procédé suivant la revendication 16, caractérisé en ce que la division axiale imperméable du manchon en chenaux axiaux s'effectue par couture, suture, scellage à chaud ou à froid, ou tout autre moyen analogue desdites deux parties périphériques opposées du manchon.

18. Procédé suivant l'une des revendications 16 et 17, caractérisé en ce que l'étape d'attachement comprend une expansion radiale du manchon à ses extrémités, à un diamètre supérieur à celui du tuteur ou des éléments de tuteur, une application d'adhésif sur la périphérie du manchon et un enfilage du tuteur ou des éléments de tuteur sur le manchon revêtu d'adhésif.

19. Procédé suivant l'une des revendications 16 et 17, caractérisé en ce que l'étape d'attachement comprend une expansion radiale du manchon à ses extrémités, à un diamètre supérieur à celui du tuteur ou des éléments de tuteur, une immersion du tuteur ou des éléments de tuteur dans un adhésif et leur enfilage sur le manchon.

20. Procédé suivant l'une des revendications 16 à 19, caractérisé en ce que le tuteur tubulaire ou les éléments de tuteur tubulaire comportent un revêtement interne et/ou externe en une matière souple, biocompatible, de préférence de même nature que la matière du manchon.

21. Procédé suivant la revendication 20, caractérisé en ce que l'étape d'attachement comprend une adhésion du tuteur tubulaire ou des éléments de tuteur tubulaire au manchon par un adhésif de même nature que la matière utilisée pour le revêtement interne et/ou externe du tuteur ou des éléments de tuteur et que celle du manchon.

## Patentansprüche

1. Rohrförmiges Schaftelement (1) einer endoluminalen Prothese für die Verzweigung von Kanälen des menschlichen oder tierischen Körpers, das radial expandierbar und komprimierbar ist und, in Expansionsstellung, in einem Hauptkanal dieser Verzweigung anzubringen ist,
dadurch gekennzeichnet, daß es einen Hohlraum (2) aufweist, der sich längs einer Längsachse zwischen zwei Enden des Schaftelements (1) erstreckt, wobei der Hohlraum (2) an einem ersten der beiden Enden durch eine erste Öffnung und an einem zweiten der beiden Enden durch eine zweite Öffnung offen ist und axial unterteilt ist und gegenüber den Körperflüssigkeiten auf wenigstens einem Teil seiner Länge undurchlässig ist, derart daß so mehrere axiale Röhren (7, 8) gebildet sind, die sich jeweils gemäß einer Achse parallel zu der Längsachse erstrecken.

2. Schaftelement einer Endoprothese gemäß Anspruch 1, dadurch gekennzeichnet, daß es die axialen Röhren (7, 8) nur auf einem mittleren Teil seines Hohlraums (2) aufweist.

3. Schaftelement einer Endoprothese gemäß einem der Ansprüche 1 und 2, dadurch gekennzeichnet, daß es zwei axiale Röhren (7, 8) aufweist.

4. Schaftelement einer Endoprothese gemäß einem der Ansprüche 1 bis 3, dadurch gekennzeichnet, daß es zwischen seinen Enden eine Hülse (4, 35) aus biokompatiblem, elastischem Material, die gegenüber den in der Verzweigung fließenden Körperflüssigkeiten undurchlässig ist und die diesen Hohlraum (2) und sein Röhren (7, 8) bildet, und wenigstens an jedem Ende des Schaftelements (1) ein radial expandierbares und komprimierbares, rohrförmiges Stützelement (9, 10), an dem die Hülse (4, 35) befestigt ist, aufweist.

5. Schaftelement einer Endoprothese gemäß einem der Ansprüche 1 bis 3, dadurch gekennzeichnet, daß es zwischen seinen Enden eine Hülse (4, 35) aus elastischem biokompatiblem Material, die gegenüber den Körperflüssigkeiten, die in der Verzweigung fließen, undurchlässig ist, und die diesen Hohlraum (2) und seine Röhren (7, 8) bildet, und eine radial expandierbare und komprimierbare, rohrförmige Stütze (3, 54), die die Hülse (4, 35) umgibt und an der diese wenigstens an den Enden des rohrförmigen Schaftelements (1) befestigt ist, aufweist.

6. Schaftelement nach einem der Ansprüche 4 und 5, dadurch gekennzeichnet, daß die Hülse (4, 35) diametral entgegengesetzte Teile (5, 6) aufweist, die gegenseitig dicht bezüglich der Körperflüssigkeiten auf wenigstens einem Teil der Länge der Hülse (4, 35) verbunden sind, und so zwei Röhren (7, 8) im Inneren der Hülse bilden.

7. Schaftelement einer Endoprothese nach einem der Ansprüche 4 bis 6, dadurch gekennzeichnet, daß die an der rohrförmigen Stütze (3, 54) oder dem oder den rohrförmigen Stützelement(en) (9, 10) befestigte Hülse eine direkte innere Umhüllung auf wenigstens einem Teil von dieser oder diesen bildet.

8. Schaftelement einer Endoprothese nach einem der Ansprüche 4 bis 6, dadurch gekennzeichnet, daß die radial expandierbare und komprimierbare, rohrförmige Stütze (3, 54) oder jedes radial expandierbare und komprimierbare, rohrförmige Stützelement (9, 10) eine innere Umhüllung (11) aus einem inerten biokompatiblen Material, das gegenüber den in der Verzweigung fließenden Körperflüssigkeiten undurchlässig ist, aufweist, und daß die Hülse (4, 35) an der Stütze (3, 54) oder an den Stützelementen (9, 10) mittels dieser inneren Umhüllung befestigt ist.

9. Schaftelement einer Endoprothese nach einem der Ansprüche 1 bis 3, dadurch gekennzeichnet, daß es zwischen seinen Enden eine radial expandierbare oder komprimierbare, rohrförmige Stütze (3, 54) aufweist, die eine Umhüllung (11, 12) aus einem biokompatiblen, gegenüber den in der Verzweigung fließenden Körperflüssigkeiten undurchlässigen Material aufweist, daß die bedeckte rohrförmige Stütze (3, 54) den Hohlraum (2) bildet, und daß das rohrförmige Schaftelement (1) außerdem wenigstens ein elastisches Trennelement aufweist, das in diesem Hohlraum (2) derart befestigt ist, daß dieser auf wenigstens einem Teil seiner Länge in die mehreren axialen Röhren (7, 8) unterteilt wird.

10. Schaftelement einer Endoprothese nach einem der Ansprüche 4 bis 9, dadurch gekennzeichnet, daß die rohrförmige Stütze (3, 54) bzw. die rohrförmigen Stützelemente (9, 10) eine innere (11) und/oder äußere Umhüllung (12) aus einem biokompatiblen, gegenüber den Körperflüssigkeiten undurchlässigen Material aufweisen.

11. Schaftelement einer Endoprothese gemäß einem der Ansprüche 5 bis 10, dadurch gekennzeichnet, daß die expandierbare, rohrförmige Stütze (3, 54), in Expansionsstellung, wenigstens ein Ende aufweist, das nach innen geht.

12. Endoluminale Prothese zur Verzweigung von Kanälen eines menschlichen oder tierischen Körpers, die ein rohrförmiges Schaftelement (1) gemäß einem der Ansprüche 1 bis 11 sowie wenigstens ein radial expandierbares und komprimierbares, rohrförmiges Abzweigungselement (13, 33) aufweist, das axial zwei Enden und einen an diesen beiden Enden offenen Hohlraum (30) aufweist, wobei jedes Abzweigungselement, in seiner Kompressionsstellung, unabhängig vom rohrförmigen Schaftelement (1) ist und, in Expansionsstellung, ein Ende, das in einer der axialen Röhren (7, 8) des rohrförmigen Schaftelements anzubringen ist, und ein anderes Ende aufweist, das außerhalb des rohrförmigen Schaftelements (1), in einem Seitenkanal der Verzweigung liegt.

13. Endoprothese nach Anspruch 12, dadurch gekennzeichnet, daß jedes rohrförmige Abzweigungselement (13, 33) zwischen seinen Enden eine Hülle aus biokompatiblem, elastischem Material, die gegenüber den durch die Verzweigung fließenden Körperflüssigkeiten undurchlässig ist und ihren oben genannten Hohlraum bildet, und, wenigstens an jedem Ende des rohrförmigen Abzweigungselements, ein radial expandierbares und komprimierbares, rohrförmiges Stützelement aufweist, an dem die Hülle befestigt ist.

14. Endoprothese nach einem der Ansprüche 12 und 13, dadurch gekennzeichnet, daß jedes rohrförmige Abzweigungselement (13, 33), zwischen seinen Enden, eine radial expandierbare und komprimierbare, rohrförmige Stütze (14) aufweist, die eine innere und/oder äußere Umhüllung (15) aus einem gegenüber Körperflüssigkeiten undurchlässigen, biokompatiblen Material aufweist.

15. Endoprothese nach einem der Ansprüche 12 bis 14, dadurch gekennzeichnet, daß die Röhren (7, 8) des rohrförmigen Schaftelements einen kreisförmigen Querschnitt aufweisen, mit einem Durchmesser der etwa gleich einem Durchmesser eines Seitenkanals dieser Verzweigung ist.

16. Verfahren zur Herstellung eines radial expandierbaren und komprimierbaren, rohrförmigen Schaftelements zum Einsatz bei einer luminalen Endoprothese für die Kanalverzweigung in einem menschlichen oder tierischen Körper, das aufweist:
- eine Bildung einer rohrförmigen Hülse aus biokompatiblem, elastischem Material, das gegenüber den in der Verzweigung fließenden Körperflüssigkeiten undurchlässig ist und die einen Hohlraum aufweist, der sich entlang einer Längsachse zwischen zwei offenen Enden der Hülse erstreckt,
- eine axiale Unterteilung des Hohlraums, auf wenigstens einem Teil seiner Länge, durch Verbinden von zwei gegenüberliegenden Randteilen der Hülse in einer gegenüber Körperflüssigkeiten undurchlässigen Weise, so daß zwei axiale Röhren gebildet werden,
- ein Anbringen dieser Hülse auf wenigstens einem Teil ihrer Länge an einer radial expandierbaren und komprimierbaren, rohrförmigen Stütze oder an wenigstens einem radial expandierbaren und komprimierbaren, rohrförmigen Stützelement.

17. Verfahren nach Anspruch 16, dadurch gekennzeichnet, daß die undurchlässige axiale Unterteilung der Hülse in axiale Röhren durch Schneiden, Nähen, Heiß- oder Kaltsiegeln oder jedes andere analoge Mittel der beiden gegenüberliegenden Randteile der Hülse durchgeführt wird.

18. Verfahren nach einem der Ansprüche 16 und 17, dadurch gekennzeichnet, daß der Schritt des Anbringens ein radiales Dehnen der Hülse an ihren Enden, auf einen Durchmesser, der größer ist als derjenige der Stütze oder des Stützelements, ein Auftragen von Klebstoff auf dem Rand der Hülse und ein Anbringen der Stütze oder der Stützelemente an der mit Klebstoff bedeckten Hülse umfaßt.

19. Verfahren nach einem der Ansprüche 16 und 17, dadurch gekennzeichnet, daß der Schritt des Anbringens ein radiales Dehnen der Hülse an ihren Enden, auf einen Durchmesser, der größer ist als derjenige der Stütze oder des Stützelements, ein Eintauchen der Stütze oder der Stützelemente in einen Klebstoff und ihr Anbringen an der Hülse umfaßt.

20. Verfahren nach einem der Ansprüche 16 bis 19, dadurch gekennzeichnet, daß die rohrförmige Stütze oder die rohrförmigen Stützelemente eine innere und/oder äußere Umhüllung aus einem biokompatiblen elastischen Material, vorzugsweise der gleichen Art wie das Material der Hülse, aufweisen.

21. Verfahren nach Anspruch 20, dadurch gekennzeichnet, daß der Schritt des Anbringens ein Kleben der rohrförmigen Stütze oder der rohrförmigen Stützelemente an die Hülse durch einen Klebstoff gleicher Art wie des Materials, das für die innere und/oder äußere Umhüllung der Stütze oder der Stützelemente verwendet wird, und wie desjenigen der Hülse, umfaßt.

## Claims

1. Tubular trunk element (1) to be used in an intraluminal prosthesis for a ramification of vessels in the human or animal body, this tubular trunk element being radially expandable and compressible and, in the expansion position, being applied in a principal vessel of the said ramification, characterized in that the tubular trunk element comprises a cavity (2) which extends along a longitudinal axis between two ends of the trunk element (1), said cavity being open at a first one of said two ends by a first opening and at a second one of said two ends by a second opening and being axially divided in a manner impermeable to the body fluids, at least over part of its length in such a way as to form several axial channels (7, 8) each of which extends along an axis parallel to said longitudinal axis.

2. Endoprosthesis trunk element according to Claim 1, characterized in that it includes the said axial channels (7, 8) uniquely on a central part of its cavity (2).

3. Endoprosthesis trunk element according to one of Claims 1 and 2, characterized in that it includes two axial channels (7, 8).

4. Endoprosthesis trunk element according to one of Claims 1 to 3, characterized in that it comprises, between its ends, a sleeve (4, 35) made of flexible, biocompatible material, which is impermeable to the body fluids passing trough the said ramidification and which forms the said cavity (2) and its channels (7, 8), and, at least at each end of the trunk element (1), a tubular stent element (9, 10) which is radially expandable and compressible and to which the sleeve (4, 35) is fixed.

5. Endoprosthesis trunk element according to one of Claims 1 to 3, characterized in that it comprises, between its ends, a sleeve (4, 35) made a flexible, biocompatible material, which is impermeable to the body fluids passing through the said ramification, and which forms the said cavity (2) and its channels (7, 8), and a tubular stent (3, 54), which is radially expandable and compressible, which surrounds the sleeve (4, 35) and on which the latter is fixed at least at the ends of the tubular trunk element (1).

6. Endoprosthesis trunk element according to one of Claims 4 and 5, characterized in that the sleeve (4, 35) has diametrally opposite parts (5, 6) which are joined to one another in a manner sealed against the body fluids over at least part of the length of the sleeve (4, 35), thereby forming two channels (7, 8) inside the sleeve.

7. Endoprosthesis trunk element according to one of Claims 4 to 6, characterized in that the sleeve (4, 35) fixed on the tubular stent (3, 54) or the tubular stent element or elements (9, 10) forms a direct internal covering over at least part of the stent or stent elements.

8. Endoprosthesis trunk element according to one of Claims 4 to 6, characterized in that the radially expandable and compressible tubular stent (3, 54), or each radially expandable and compressible tubular stent element (9,10), comprises an internal covering (11) made of a biocompatible inert material, which is impermeable to the body fluids passing through the said ramification, and in that the sleeve (4, 35) is fixed to the stent (3, 54) or to the stent elements ( 9, 10) by way of this internal covering.

9. Endoprosthesis trunk element according to one of Claims 1 to 3, characterized in that it comprises, between its ends, a radially expandable or compressible tubular stent (3, 54) which has a covering (11, 12) made of a biocompatible material, which is impermeable to the body fluids passing through the said ramification, in that the covered tubular stent (3, 54) forms the said cavity (2) and in that the tubular trunk element (1) additionally comprises at least one flexible partition element, fixed in the said cavity (2) in such a way as to divide the latter, over at least part of its length, into the said several axial channels (7, 8).

10. Endoprosthesis trunk element according to any one of Claims 4 to 9, characterized in that the tubular stent (3, 54) or, respectively, the tubular stent elements (9, 10) have an internal covering (11) and/or external covering (12) made of a biocompatible material which is impermeable to the body fluids.

11. Endoprosthesis trunk element according to one of Claims 5 to 10, characterized in that the expandable tubular stent (3, 54) has, in the expansion position, at least one end which widens outwards.

12. Intraluminal prosthesis for ramification of vessels in a human or animal body, comprising a tubular trunk element (1) according to any one of Claims 1 to 11, as wells as at least one tubular branch element (13, 33) which is radially expandable and compressible and which axially has two ends and a cavity (30) open at these two ends, each branch element being, in its compression position, independent of the tubular trunk element (1), and having an end which is to be applied, in the expansion position, within one of the said axial channels (7, 8) of the tubular trunk element, and another end situated outside the tubular trunk element (1), within a secondary vessel of the said ramification.

13. Intraluminal prosthesis according to Claim 12, characterized in that each tubular branch element (13, 33) comprises, between its ends, a sheath made of a flexible, biocompatible material which is impermeable to the body fluids passing through the said ramification and which forms its abovementioned cavity (30) and, at least at each end of the tubular branch element, a tubular stent element which is radially expandable and compressible and on which the sheath is fixed.

14. Intraluminal prosthesis according to one of Claims 12 or 13, characterized in that each tubular branch element (13, 33) comprises, between its ends, a tubular stent (14) which is radially expandable and compressible and which has an internal and/or external covering (15) made of a biocompatible material which is impermeable to the body fluids.

15. Intraluminal prosthesis according to any one of Claims 12 or 14, characterized in that the channels (7, 8) of said tubular trunk element have a circular cross-section with a diameter which is approximately equal to the diameter of a secondary vessel of the said ramification.

16. Method for producing a radially expandable and compressible tubular trunk element to be used in an intraluminal prosthesis for a ramidification of vessels in the human or animal body, comprising
- formation of a tubular sleeve made of a flexible and biocompatible material, which is impermeable to the body fluids passing through the said ramification, and which has a cavity which extends along a longitudinal axis between two open ends of the sleeve,
- axial partition of said cavity, over at least part of its length, by joining in a manner impermeable to the body fluids, two opposite peripheral parts of the sleeve, in such a way as to form two axial channels,
- attachment of this sleeve, over at least part of its length, to a radially expandable and compressible tubular stent or to at least one radially expandable and compressible tubular stent element.

17. Method according to Claim 16, characterized in that the leaktight axial partition of the sleeve in axial channels is obtained by seaming, stitching, heat sealing or cold sealing, or any other similar means, said two opposite peripheral parts of the sleeve.

18. Method according to one of Claims 16 and 17, characterized in that the attachment step comprises radially expanding the sleeve, at its ends, to a diameter greater than that of the stent or of the stent elements, applying adhesive to the periphery of the sleeve and engaging the stent or the stent elements on the sleeve coated with adhesive.

19. Method according to one of Claims 16 and 17, characterized in that the attachment step comprises radially expanding the sleeve, at its ends, to a diameter greater than that of the stent or of the stent elements, immersing the stent or the stent elements in an adhesive and engaging them on the sleeve.

20. Method according to one of Claims 16 to 19, characterized in that the tubular stent or the tubular stent elements include an internal and/or external covering made of a flexible and biocompatible material, preferably of the same nature as the material of the sleeve.

21. Method according to Claim 20, characterized in that the attachment step comprises adhesion of the tubular stent or of the tubular stent elements to the sleeve by means of an adhesive of the same nature as the material used for the internal and/or external covering of the stent or of the stent elements and of the same nature as the material of the sleeve.
